# EUROPEAN PATENT APPLICATION

(11) **EP 2 339 018 A2**
(43) Date of publication of application: **29.06.2011**
(21) Application number: 10192290.4
(22) Date of filing: 23.11.2010
(51) Int. Cl.: C12Q 1/04, C12M 1/00

(54) **Staining process and apparatus used in bacteriology**

(30) Priority: 26.11.2009 BR 09046909
(71) Applicant: Hemogram Ind. Com. Prods Hospitalares LTDA., CEP 12908-829 Brasil (BR)
(72) Inventor: Luiz Resende de Castro, Eduardo, CEP 12916-030 Brasil (BR)
(74) Representative: Zimmermann, Tankred Klaus

(57) **Abstract**

The present invention refers to a staining process used in bacteriology able to standardize and make Gram's staining procedure easier and faster, preventing variations in its process.

It further refers to an apparatus able to perform said process.

The process comprises different placing steps, with different reagents and times of contact with the sample plates.

The apparatus comprises peristaltic pumps to provide each reagent, sensors to indicate plate presence, switches to indicate the time the pumps were operating, a motor along with an external gear set to make the plate move for the staining processes, fan to help drying the plate after the process, buttons for starting and turning circuit on and lamps for machine in use and lack of reagent indications, as well as a reagent draining tank.

## Description

The present invention refers to a staining process, used in bacteriology, that is able to standardize and render the Gram's staining procedure easier and faster, preventing variations in its process.

It further refers to an apparatus that is able to perform said process.

### INTRODUCTION

Most stains used in bacteriology are intended to the fast presumptive diagnosis of the infectious process and also previous assessment of the sample quality.

Gram's staining is the most important bacterioscopic method currently most used in bacteriology, and it is intended to classify microorganisms based on its staining characteristics, size, shape and cell arrangement.

Bacteria are classified basically in two large groups: Gram-positive and Gram-negative.

Regarding the staining characteristics, Gram-positive bacteria stain purplish blue and Gram-negative bacteria stain pink.

Detection of microorganisms in the sample is crucial, as it may indicate an antimicrobial therapy guided to a certain group of diseases, and also the need to provide culture means with diagnosis and/or epidemiological purposes.

### STAINING PROCESS

In order to establish the mechanism involved in the staining method, several concepts have been provided, such as:
- Gram-positive and gram-negative bacteria would have different affinities with primary stain violet crystal;
- The existence of different grades of permeability on the Gram-positive and Gram-negative microorganisms wall.

Both cell wall thickness and interstitial spaces dimensions, e.g., "pore diameter", seem to be decisive to the final Gram's staining result.

According to that concept, when the cell structures are covered by Violet Crystal, all of them stain purple. After that process the plate receives a cleaning solution to remove the excess of Violet Crystal. By adding Lugol, also called mordant, iodine-pararosaniline complex is formed. This reaction has the property of fixing the primary stain to the stained structures. Some structures lose violet color fast, when a destaining agent is applied, such as ethyl alcohol (destaining solution), while others lose their color more slowly or not at all. Fuchsine and saphranine stain destained structures.

Bacteria having their cell wall composed of murein (n-acetyl-muramic acid peptidoglycan), during the destaining process with ethyl alcohol, retain the stain. Bacteria having their cell walls composed mostly of fatty acids (lipopolysaccharides and lipoproteins, on the other hand, lose iodine-pararosaniline complex, taking over the color of the counterstain.

### DESCRIPTION OF THE PRIOR ART

Staining processes known in the current art have some drawbacks.

The Gram's staining technique usually follows an action protocol standardizing it. However, there are variations in that procedure, related to staining performance time and to the use of water washing in certain steps, which may or may not interfere with stained structures viewing.

One problem in the current art refers to the fact that it is performed manually, being thus, susceptible to several external factors that may affect the process.

In the manual process, the contact time between the reagent and the staining plate is not accurate.

As it is performed manually, the process depends on the intervention of an operator to perform it. Thus, excessive reagent application may also occur for a single sample, and consequently, reagent waste and changed results.

In the manual process known in the current art, there is direct contact of the operator and the plate in the process medium.

Further, as it is a manual process, there is the possibility of mistaken staining and of the blades being wet at the end of the staining process.

### OBJECTS OF THE INVENTION

The staining process and the respective apparatus to perform it are intended to standardize and make the Gram's staining procedure easier and faster, preventing variations in its process, standardizing the procedure as follows:
- Amount of each stain in the sample;
- Amount of cleaning solution in the sample;
- Amount of destaining agent in the sample;
- Contact time of stain and sample;
- Contact time of destaining agent and sample;
- Completion time for each sample.

Thus, the current art variation is avoided in the manual process. Thus, we have plates with homogeneous staining, improving analysis and diagnosis of the cells analyzed.

The staining process in the present invention is performed using an apparatus through which a standardized time is obtained for the contact of reagent and plate.

Another advantage of the present invention refers to the fact that the process depends only on feeding the plates into the staining apparatus by an operator, and there is no contact by him/her while the process is in progress.

Further, the apparatus makes the application of the reagent required for each sample, preventing reagent waste in the process.

As it is an automated process, there is no possibility of a staining mistake, resulting in a homogeneous staining, and also, the plates come dry at the end of the staining process.

### BRIEF DESCRIPTION OF THE DRAWINGS

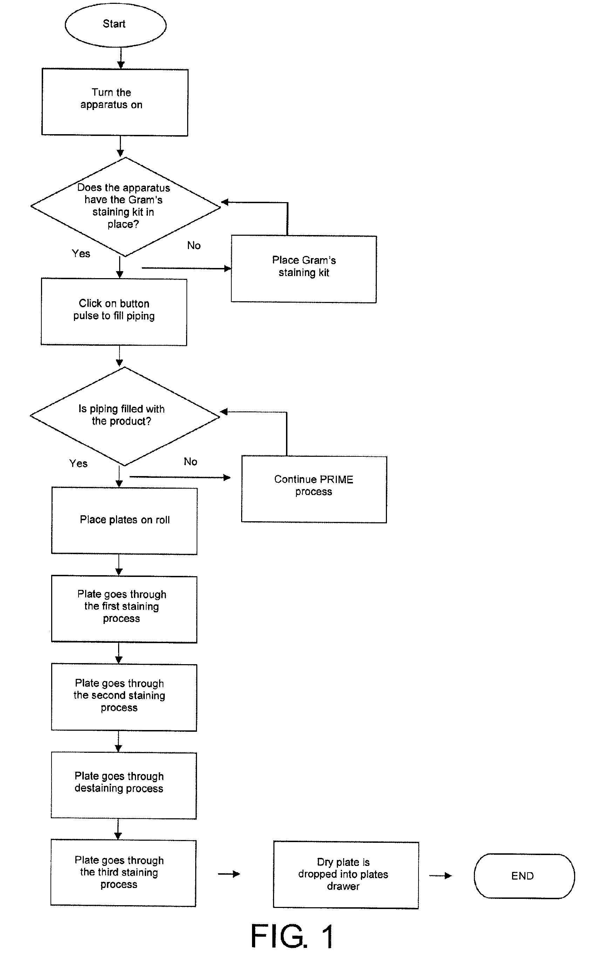

Figure 1 shows a block diagram indicating the staining process steps in the present invention, performed by a staining apparatus.

### DETAILED DESCRIPTION OF THE INVENTION

The present invention refers to a staining process used in bacteriology, the purpose of which is to perform Gram's staining in an automated way, by means of an apparatus for performing it, and said process comprises the following steps:
- feeding the staining apparatus with the Gram's staining kit;
- filling apparatus piping with the reagents;
- introducing the plates to be stained into the staining apparatus roll;
- first plates staining;
- removing excess reagent;
- second plates staining;
- plates destaining;
- third plates staining;
- deployment of already dry plates into plates drawer.

The staining process is performed by means of an apparatus comprising peristaltic pumps to provide each reagent, sensors to indicate plate presence, switches to indicate the time the pumps are operating, a motor along with an external gear set to make the plate move for the staining processes, fan to help drying the plate after the process, buttons for starting and turning circuit on and lamps for machine in use and lack of reagent indications.

Five is the number of reagents composing the staining kit used by the apparatus:
1. Violet Crystal : primary stain, stains cytoplasm crimson, regardless the cell type.
2. Cleaning solution: It is intended only to remove excess of Crystal Violet.
3. Iodine-Lugol Solution: Mordant - increases affinity between crystal violet and the cell, and forms, along with the stain, an insoluble complex inside the cell.
4. Destaining Solution - Ethyl alcohol, acetone or both: lipidic solvent.
5. Saphranine Solution / Fuchsine Solution: Contrasting stain - basic saphranine or fuchsine: stains cytoplasm red from gram-negative cells that have been destained.

When turning the apparatus on, the plates start moving, by means of worm thread shafts coupled into a gear set, and one of them is held to an AC, 1 RPM motor. With the plate moving, it reaches the first point, where activates the first presence sensor, the first reagent pump, Crystal Violet, fills the plate with the reagent. After about 1 minute with the reagent in contact with the plate, that reagent runs through a slot on the table, and goes to the Cleaning Solution, which operates on a continuous basis while the plate passes, removing only the excess primary stain and running through another slot on the table, the plate goes then to the second reagent, Lugol, which also fills the plate for about 1 minute as soon as the third presence sensor is activated. The reagent is run again by means of a slot on the table. When activating the fourth presence sensor, the pump starts injecting Absolute Alcohol, on a continuous basis, operating during almost all the time the plate is passing.

At last it gets to Saphranine or Fuchsine, which also fills the whole plate, only the contact with the plate is shorter, only 30 seconds, when activating the fifth presence sensor, and after that is also runs through a lowering on the table.

Each of the sensors above the table operates serially with the operating time sensor in the gear, in such a way that only when both sensors are activated, the pump control board sends a signal for the pump to be started.

The apparatus has an on/off lamp, which is turned on in parallel with the button to start the machine, in such a way that when the button is pressed and the machine is started, the light shall always be lit. The level lamp, on the other hand, is connected serially with the tray sensor, which is activated or not by means of the thrust spring, in such a way that the less reagent there is, the lighter the tray gets, activating the sensor and turning the lamp on.

Piping feeding with reagents is started by means of a Pulse key, and the voltage goes right to the plates and makes the pumps work all at the same time, filling the hose with the reagents and getting the machine ready to operate.

The apparatus is provided with a sliding transportation system, having moving spirals. There are two parallel spirals with opposite rotations moving the plate along the table, and also controlling the plate moving time.

The table encompasses all the space between spirals. There are raised guides to make plate moving easier during the staining process. It is made of stainless steel and molded design to control reagents input and output.

The draining tank is located on the front of the apparatus, right under the table, and it is intended to store the excess reagent used in the process.

The apparatus is provided with five pumps, one for each reagent. Each pump has its own board for controlling functions.

Plates presence sensors are activated by the plate itself, when it reaches a certain point after the stand-by time scheduled. They operate by releasing the reagent, and each pump has a sensor located on the upper part of the table.

The apparatus also comprises a plate container, to store them after the staining process.

## Claims

1. **"STAINING PROCESS USED IN BACTERIOLOGY", characterized in that** it comprises the following steps:
- feeding the staining apparatus with the Gram's staining kit;
- filling apparatus piping with the reagents;
- introducing the plates to be stained into the staining apparatus roll;
- first plates staining;
- removing excess reagent;
- second plates staining;
- plates destaining;
- third plates staining;
- deployment of already dry plates into plates drawer.

2. **"PROCESS"**, as claimed in claim 1, **characterized in that** the reagent used in the first step of the plates staining process is Crystal Violet.

3. **"PROCESS"**, as claimed in claim 1, **characterized in that** the reagent used for removing excess reagent from plates is the Cleaning Solution.

4. **"PROCESS"**, as claimed in claim 1, **characterized in that** the reagent used in the second step of plates staining is Iodine-Lugol solution.

5. **"PROCESS"**, as claimed in claim 1, **characterized in that** the destaining solution used for plates destaining is ethyl alcohol, acetone, or both.

6. **"PROCESS"**, as claimed in claim 1, **characterized in that** the reagent used in the third step of plates staining is saphranine or fuchsine solution.

7. **"PROCESS"**, as claimed in claim 1, **characterized in that** the reagent in the first plates staining is in contact with them for 1 minute.

8. **"PROCESS"**, as claimed in claim 1, **characterized in that** the reagent in the second plates staining is in contact with them for 1 minute.

9. **"PROCESS"**, as claimed in claim 1, **characterized in that** the reagent in the third plates staining is in contact with them for 30 seconds.

10. **"PROCESS"**, as claimed in claim 1, **characterized in that** plates are dried by means of a drying system composed of a fan, which guides the air flow, operating on a continuous basis.

11. **"APPARATUS FOR PLATES STAINING", characterized in that** it comprises peristaltic pumps to provide each reagent, sensors to indicate plate presence, switches to indicate the time the pumps were operating, a motor along with an external gear set to make the plate move for the staining processes, fan to help drying the plate after the process, buttons for starting and turning circuit on and lamps for machine in use and lack of reagent indications, as well as a reagent draining tank.

12. **"APPARATUS"**, as claimed in claim 11, **characterized in that** the plates move by means of worm thread shafts coupled to a gear set.

13. **"APPARATUS",** as claimed in claim 11, **characterized in that** each sensor over the table operates serially with the operating time sensor in the gear, in such a way that only when both sensors are activated, the pump control board sends a signal for the pump to be started.

14. **"APPARATUS",** as claimed in claim 11, **characterized in that** piping feeding with reagents is started by means of a Pulse key, and the voltage goes right to the plates and makes the pumps work all at the same time, filling the hose with the reagents and getting the machine ready to operate.

15. **"APPARATUS"**, as claimed in claim 11, **characterized in that** it comprises a sliding transportation system, having moving spirals. There are two parallel spirals with opposite rotations moving the plate along the table, and also controlling the plate moving time.

16. **"APPARATUS"**, as claimed in claim 15, **characterized in that** the table encompasses all the space between spirals, having raised guides to make plate moving easier during the staining process.

17. **"APPARATUS"**, as claimed in claim 16, **characterized in that** said table is made of stainless steel alloy and molded design to control reagents input and output.

18. **"APPARATUS"**, as claimed in claim 11, **characterized in that** the draining tank is located on the front of the apparatus, right under the table, storing the excess reagent used in the process.

19. **"APPARATUS"**, as claimed in claim 11, **characterized in that** it comprises five pumps, one for each reagent, and each pump has its own board for controlling functions.

20. **"APPARATUS"**, as claimed in claim 11, **characterized in that** plates presence sensors are activated by the plate itself, when it reaches a certain point after the stand-by time scheduled.

21. **"APPARATUS"**, as claimed in claim 11, **characterized in that** it comprises a plate container storing them after the staining process.

22. **"APPARATUS",** as claimed in claim 11, **characterized in that** it comprises an on/off lamp, which is turned on in parallel with the button to start the machine.

23. **"APPARATUS"**, as claimed in claim 11, **characterized in that** it comprises a level lamp connected serially with the tray sensor, which is activated or not by means of the thrust spring, in such a way that the less reagent there is, the lighter the tray gets, activating the sensor and turning the lamp on.
